## Europäisches Patentamt

## European Patent Office

## Office européen des brevets

(11) Publication number: **0 208 024**
**B1**

# EUROPEAN PATENT SPECIFICATION

(45) Date of publication of patent specification: **29.11.89**

(51) Int. Cl.⁴: **B 07 C 5/36,** G 01 N 33/08

(21) Application number: **85201143.6**

(22) Date of filing: **08.07.85**

(54) **Apparatus for sorting and removing undesirable objects from a feed belt conveyor.**

(43) Date of publication of application:
**14.01.87 Bulletin 87/03**

(45) Publication of the grant of the patent:
**29.11.89 Bulletin 89/48**

(84) Designated Contracting States:
**DE GB NL**

(56) References cited:
EP-A-0 092 989
EP-A-0 098 733
DE-A-3 117 464
DE-C- 595 608
GB-A-1 112 823
GB-A-2 119 335
NL-A-8 400 080
US-A-2 112 259
US-A-3 119 457
US-A-3 901 334
US-A-4 164 291

(73) Proprietor: **Staalkat B.V.**
**Ambachtsstraat 4**
**NL-7122 MP Aalten (NL)**

(72) Inventor: **van der Schoot, Jelle**
**Spinkatstraat 9**
**NL-7121 AL Aalten (NL)**

(74) Representative: **Smulders, Theodorus A.H.J., Ir.**
**et al**
**Vereenigde Octrooibureaux Nieuwe Parklaan**
**107**
**NL-2587 BP 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to an apparatus for sorting fragile objects supplied by a feed conveyor and for removing those objects which are defective, the apparatus comprising, in addition to the feed conveyor, an inspection station arranged along said feed conveyor, a discharge means downstream of said feed conveyor, separator means for removing defective objects at the downstream end of the feed conveyor, a tracking memory arranged to store data defining the position on the feed conveyor of objects which are identified as defective at the inspection station and to actuate the separator means when the defective object arrives thereat.

Such an apparatus is known from US-A-4,164,291. Although in this patent it is said that the described apparatus is also useful for sorting and removing eggs and the like vulnerable products, it has not been made clear. In the described apparatus the removing of unwanted products takes place with the help of ejector pins, which are entirely unsuitable for handling eggs.

It is an object of the present invention to eliminate the above drawback.

To this effect an apparatus of the above described type adapted in particular for processing vulnerable objects such as eggs, is characterized in that the discharge means comprises a discharge conveyor aligned with the feed conveyor with regard to the direction of object transport, said separator means consists of a trapdoor arranged between the feed and the discharge conveyors so as to enable dropping of an object passing the trapdoor, above the trapdoor there is arranged a turnstile by means of which objects not causing actuation of the trapdoor are transferred from the feed conveyor to the discharge conveyor, downstream of the trapdoor there are provided weighing means for sorting objects supplied from the discharge conveyor with regard to weight.

However, it is also possible that the discharge means comprises a discharge conveyor aligned with the feed conveyor with regard to the direction of object transport, the discharge conveyor comprises base means for supporting the objects along said direction of transport and a double chain mechanism arranged above said base means so as to transfer the objects from the feed conveyor to the base means and to convey the objects along the base means, said double chain mechanism including two spaced chains and bars fixedly mounted to and extending between the two chains so as to enter into contact with the objects for transferring and conveying them, said separator means consists of a trapdoor arranged between the feed and the discharge conveyors so as to enable dropping of an object passing the trapdoor, downstream of the trapdoor there are provided weighing means for sorting the objects supplied from the discharge conveyor with regard to weight.

The trapdoor can be realized either by a flap disposed in the egg discharge path, which flap is attached at the upstream end rotatably to a frame or the like, or downstream of the trapdoor a hare's jump, or a fork-shaped member movable vertically up and down, whose prongs during their movement can pass along the teeth of a guideplate arranged at an acute angle to the vertical.

For the sake of completeness it is observed that British patent No. 1,112,823 describes an apparatus for sorting and removing unwanted eggs, in which the eggs are discharged laterally from the candling station, so before the weighing station. The discharge takes place via a belt, and via a following belt. However, the eggs then move over the first part of a straight discharge belt for wanted eggs; this implies that when a broken egg loses its contents, also a part of the belt for discharge of wanted eggs will be fouled, with all consequences resulting therefrom.

Further US patent 2,112,259 describes an egg grading machine in which use is made of a double chain mechanism including two spaced chains and bars fixedly mounted to and extending between the two chains so as to enter into contact with the objects for transferring and conveying them. However, this apparatus exclusively relates to the candling of eggs and the grading for weight. The apparatus contains no trapdoor.

It may follow from above that none of the literature references cited applies the idea of the invention according to which unwanted products are removed automatically out of the track directly after candling, and especially before weighing.

Some embodiments of the apparatus for sorting and removing undesirable eggs from a feed belt conveyor will now be described, by way of example, with reference to the accompanying drawings, in which:

Fig. 1 is diagrammatic sideview of an apparatus for sorting and removing undesirable eggs from a feed belt conveyor;

Fig. 2 is a corresponding view of a part of a different apparatus for sorting and removing undesirable eggs from a belt conveyor; and

Figs. 3 shows a similar view as Figs. 2, but of a third embodiment.

As shown in the drawings, wherein the same parts are indicated by the same reference numerals, an apparatus for sorting and removing undesirable eggs from a belt conveyor comprises an only partly shown belt conveyor 1 having rollers 2, which endless belt conveyor is passed about a chain wheel 3. At the lower end there is provided a diagrammatically shown belt conveyor support wheel 4. Downstream of the chain wheel 3, through which eggs 5 are transported in the direction indicated by an arrow, there is provided a guideplate 6 with a superimposed turnstile 25 and subsequently a trapdoor or trap element 7.

The trap element 7 is designed at the top as a fork-shaped carrier 8 which is attached to the one end of a rod 9 whose other end is operated by a piston-and-cylinder assembly, not shown. The fork-shaped carrier 8 with the rod 9 are movable in the direction indicated by an arrow along a guideplate 10 consisting of teeth. Guideplate 10 is connected at its one end to a trough 11, under which there is arranged a receptacle 12, in the present case a bucket.

Downstream of the trap element 7 there is provided a conveying element 13 adapted to perform a reciprocating and an up and down movement in the form of a "hare's jump". In the drawing dotted lines indicate the movement patterns of two mounting shafts 14 of the conveying element 13 relatively to the frame, not shown. As further shown in the drawing, the frame has a recess 15 through which extends the top end of a scale 16. The scale may be of the type as described in US patent 3,980,147 of applicants.

Downstream of the conveying element 13 there is arranged a guideplate 17.

In order to detect the undesirable eggs, there is arranged underneath the top part of the belt conveyor 1, a bin 18 having a light source 19 as well as a coil system 20 feeding a "tracking" memory, not shown.

The apparatus furthermore comprises a pointer 21 having a grip 22. The one end of the pointer is provided with a head 23 having a switch and an amplifier. The other end of the pointer is fitted with a conductor 24 for connection to a current source, not shown.

The details of such an apparatus are described in Dutch patent application 7707946 and the corresponding US patent 4,164,291, of which applicants are licensees.

It will be clear that the operation of the piston-and-cylinder assembly or similar driving mechanism operating the rod 9 of the trap element 7, is controlled by the "tracking" memory, not shown, which is fed by the coil system 20.

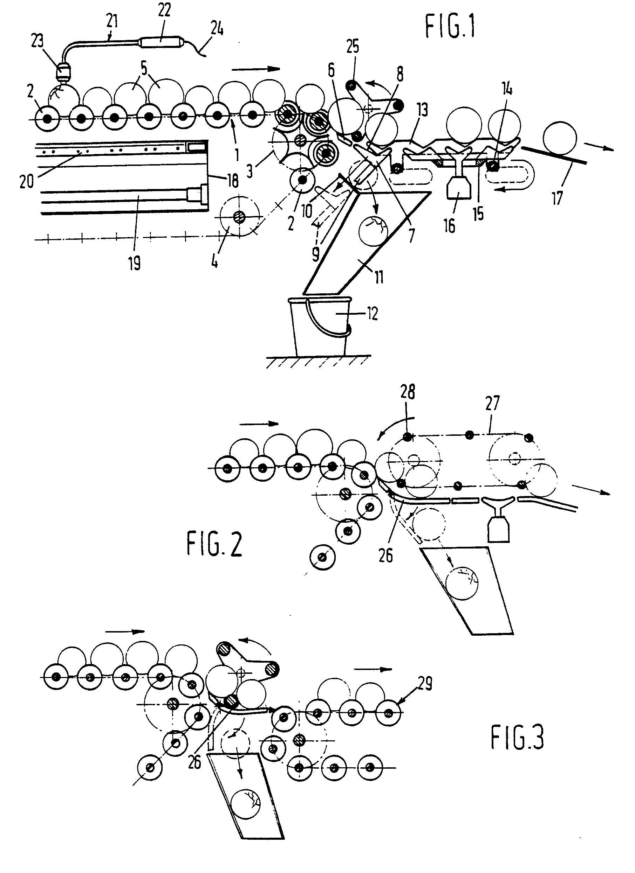

The operation of the apparatus, after the above, seems obvious. When the operator during the passage of the eggs along the top of the receptacle or bin 18 having a light source 19 detects an undesirable egg, he indicates this with the head 23 of the pointer 21. The signal produced is taken up by one of the coils 20 in the tracking memory, not shown, so that, when the further transported egg reaches the trap element 7, this is operated and moved to the position shown in dashed lines, so that the egg is discharged. As shown in Fig. 1, the eggs are moved from the belt conveyor 1 via the guideplate 6 by means of the turnstile 25 to the trap element 7. When the egg need not be removed, the trap element remains in the position indicated in full lines, so that the egg can be further transported by means of the conveying element 13 towards the scale 16 and subsequently can be discharged via the guideplate 17.

The diagrammatic representation of a second embodiment only partly shown in Fig. 2 differs in this respect from the apparatus shown in Fig. 1 in that the trap element here consists of a flap 26 connected at the upstream end rotatably to a frame, not shown. Furthermore, instead of the conveying element 13 and the turnstile 25, use is made herein of a conveying system comprising a pair of chains 27 between which there are arranged carrier elements 28.

The third embodiment shown in Fig. 3 similarly as represented in Fig. 2, comprises in the same manner as shown in Fig. 2, a flap 26 connected at its upstream end rotatably to a frame. Above the flap there is arranged similarly as in the embodiment shown in Fig. 1, a turnstile 25. Downstream of the flap, there is provided subsequently a discharge belt conveyor 29, which may be designed similarly as the belt conveyor 1.

## Claims

1. An apparatus for sorting fragile objects supplied by a feed conveyor and for removing those objects which are defective, the apparatus comprising, in addition to the feed conveyor (1),

an inspection station (18-24) arranged along said feed conveyor,

a discharge means (13) downstream of said feed conveyor,

separator means (7, 26) for removing defective objects at the downstream end of the feed conveyor,

a tracking memory arranged to store data defining the position on the feed conveyor of objects which are identified as defective at the inspection station and to actuate the separator means when the defective object arrives thereat,

characterized in that

the discharge means comprises a discharge conveyor (13) aligned with the feed conveyor (1) with regard to the direction of object transport, said separator means consists of a trapdoor (7, 26) arranged between the feed and the discharge conveyors so as to enable dropping of an object passing the trapdoor,

above the trapdoor there is arranged a turnstile (25) by means of which objects not causing actuation of the trapdoor are transferred from the feed conveyor to the discharge conveyor,

downstream of the trapdoor there are provided weighing means (16) for sorting objects supplied from the discharge conveyor with regard to weight.

2. An apparatus for sorting fragile objects supplied by a feed conveyor and for removing those objects which are defective, the apparatus comprising, in addition to the feed conveyor (1),

an inspection station (18-24) arranged along said feed conveyor,

a discharge means (13) downstream of said feed conveyor,

separator means (7, 26) for removing defective objects at the downstream end of the feed conveyor,

a tracking memory arranged to store data defining the position on the feed conveyor of objects which are identified as defective at the inspection station and to actuate the separator means when the defective object arrives thereat,

characterized in that
the discharge means comprises a discharge conveyor aligned with the feed conveyor with regard to the direction of object transport,

the discharge conveyor comprises base means for supporting the objects along said direction of transport and a double chain mechanism arranged above said base means so as to transfer the objects from the feed conveyor to the base means and to convey the objects along the base means, said double chain mechanism (27, 28) including two spaced chains (27) and bars (28) fixedly mounted to and extending between the two chains so as to enter into contact with the objects for transferring and conveying them,

said separator means consists of a trapdoor (26) arranged between the feed and the discharge conveyors so as to enable dropping of an object passing the trapdoor,

downstream of the trapdoor there are provided weighing means for sorting the objects supplied from the discharge conveyor with regard to weight.

3. An apparatus according to claim 1 or 2, characterized in that the trapdoor comprises a flap (26) disposed in the objects discharge path, which flap is attached at the upstream end rotatably to a frame or the like.

4. An apparatus according to claim 1, characterized in that downstream of the trapdoor there is arranged a hare's jump (13).

5. An apparatus according to claim 1, characterized in that the trapdoor comprises a fork-shaped member (7) movable vertically up and down, whose prongs during their movement can pass along the teeth of a guideplate (10) arranged at an acute angle to the vertical.

6. An apparatus according to claim 5, characterized in that the guideplate (10) is connected to a discharge trough (11).

7. An apparatus according to any one of the preceding claims, characterized in that transfer plates are disposed between the various conveyors arranged in the path of the objects.

**Patentansprüche**

1. Sortiervorrichtung für zerbrechliches Gut mit einem Beschickungsförderer (1),
einer längs des Beschickungsförderers angeordneten Kontrollstation (18 bis 24),
einem Entlademittel stromabwärts des Beschickungsförderers,
einem an dem stromabwärts liegenden Ende des Beschickungsförderers angeordneten Abscheider (7, 26) für fehlerhafte Teile,
einem die Positionsdaten der an der Kontrollstation als fehlerhaft erkannten Teile auf dem Beschickungsförderer aufnehmenden Spurspeicher, der den Abscheider bei der Ankunft fehlerhafter Teile auslöst,
dadurch gekennzeichnet,
daß das Entlademittel aus einem in Förderrichtung auf den Beschickungsförderer (1) ausgerichteten Entladeförderer (13) besteht,

daß der Abscheider aus einer zwischen dem Beschickungs- und Entladeförderer angeordneten Falltür (7, 26) besteht, die das Abwerfen von passierenden Teilen erlaubt,
daß oberhalb der Falltür ein Drehkreuz (25) angeordnet ist, das die Falltür nicht auslösende Güter vom Beschickungs- zum Entladeförderer transportiert,
und daß in Förderrichtung hinter der Falltür eine Wiegestation (16) zum gewichtsabhängigen Sortieren der vom Entladeförderer geförderten Teile vorgesehen ist.

2. Sortiervorrichtung für zerbrechliches Gut mit einem Beschickungsförderer (1),
einer längs des Beschickungsförderers angeordneten Kontrollstation (18 bis 24),
einem Entlademittel (13) stromabwärts des Beschickungsförderers,
einem an dem stromabwärts liegenden Ende des Beschickungsförderers angeordneten Abscheider (7, 26) für fehlerhafte Teile,
einem die Positionsdaten der an der Kontrollstation als fehlerhaft erkannten Teile auf dem Beschickungsförderer aufnehmenden Spurspeicher, der den Abscheider bei der Ankunft fehlerhafter Teile auslöst,
dadurch gekennzeichnet,
daß das Entlademittel aus einem in Förderrichtung auf den Beschickungsförderer ausgerichteten Entladeförderer besteht,
daß der Entladeförderer aus einer Auflage für das Gut längs des Förderweges und einem oberhalb der Auflage angeordneten Doppelkettenmechanismus besteht, der das Gut von dem Beschickungsförderer zur Auflage transportiert und längs dieser fördert, wobei dieser Doppelkettenmechanismus (27, 28) zwei im Abstand voneinander angeordnete Ketten (27) und sich zwischen den beiden Ketten erstreckende, daran befestigte Stangen (28) umfaßt, die mit den Gutteilen in Berührung gelangen,
daß der Abscheider aus einer zwischen dem Beschickungs- und dem Entladeförderer angeordneten Falltür (26) besteht, die das Abwerfen von passierenden Teilen erlaubt,
und daß stromab der Falltür eine Wiegestation zum gewichtsabhängigen Sortieren der durch den Entladeförderer geförderten Teile vorgesehen ist.

3. Sortiervorrichtung nach Anspruch 1 oder 2, dadurch gekennzeichnet, daß die Falltür aus einer im Gutstrom liegenden Klappe (26) besteht, die an ihrem stromaufwärts gelegenen Ende drehbar an einem Rahmen od.dgl. befestigt ist.

4. Sortiervorrichtung nach Anspruch 1, dadurch gekennzeichnet, daß stromab der Falltür ein Hubbalkenförderer (13) angeordnet ist.

5. Sortiervorrichtung nach Anspruch 1, dadurch gekennzeichnet, daß die Falltür aus einer in vertikaler Richtung auf- und abbewegbaren Gabel (7) besteht, deren Zinken während ihrer Bewegung längs der Zähne einer in einem spitzen Winkel zur Senkrechten angeordneten Führungsplatte (10) passieren können.

6. Sortiervorrichtung nach Anspruch 5, dadurch gekennzeichnet, daß die Führungsplatte (10) mit einem Entladetrog (11) verbunden ist.

7. Sortiervorrichtung nach einem oder mehreren der Ansprüche 1 bis 6, dadurch gekennzeichnet, daß Transportplatten zwischen den verschiedenen, im Förderweg des Gutes liegenden Förderern angeordnet sind.

**Revendications**

1. Un appareil pour trier des objets fragiles fournis par un transporteur d'alimentation et pour enlever les objets défectueux, cet appareil comprenant, outre le transporteur d'alimentation (1): une station d'inspection (18-24) arrangée le long dudit transporteur d'alimentation, un dispositif de décharge (13) en aval dudit transporteur d'alimentation, un dispositif de séparation (7, 26) pour enlever des objets défectueux au bout d'aval du transporteur d'alimentation, une mémoire de dépistage pour mémoriser des données définissant la position sur le transporteur d'alimentation d'objets identifiés comme défectueux à la station d'inspection et pour actionner le dispositif de séparation quand l'objet défectueux arrive à cet endroit, caractérisé en ce que le dispositif de décharge comprend un transporteur de décharge (13) aligné sur le transporteur d'alimentation (1) par rapport à la direction de transport d'objets, ledit dispositif de séparation comprend une trappe (7, 26) arrangée entre les transporteurs d'alimentation et de décharge pour permettre la chute d'un objet passant la trappe, on a arrangé audessus de la trappe un tourniquet (25) au moyen duquel des objets ne causant pas un actionnement de la trappe sont transférés du transporteur d'alimentation au transporteur de décharge, on a prévu en aval de la trappe un dispositif de pesage (16) pour trier des objets fournis du transporteur de décharge par rapport au poids.

2. Un appareil pour trier des objets fragiles fournis par un transporteur d'alimentation et pour enlever les objets défectueux, cet appareil comprenant, outre le transporteur d'alimentation (1): une station d'inspection (18-24) arrangeé le long dudit transporteur d'alimentation, un dispositif de décharge (13) en aval dudit transporteur d'alimentation, un dispositif de séparation (7, 26) pour enlever des objets défectueux au bout d'aval du transporteur d'alimentation, une mémoire de dépistage pour mémoriser des données definissant la position sur le transporteur d'alimentation d'objets identifiés comme défectueux à la station d'inspection et pour actionner le dispositif de séparation quand l'objet défectueux arrivé a cet endroit, caractérisé en ce que le dispositif de décharge, comprend un transporteur de décharge aligneé sur le transporteur d'alimentation par rapport à la direction de transport d'objets, le transporteur de décharge comprend un dispositif de base pour supporter les objets le long de ladite direction de transport et un mecanisme de chaîne double arrangeé au-dessus dudit dispositif de base pour transférer les objets du transporteur d'alimentation au dispositif de base et pour transporter les objets le long du dispositif de base, le dit mécanisme de chaîne double (27, 28) comprenant deux chaînes espacées (27) et des barres (28) monteés fixement sur, et s'etendant entre, les deux chaînes pour entrer en contact avec les objets pour les transferer et transporter, ledit dispositif de séparation comprend un trappe (26) arrangée entre les transporteurs d'alimentation et de décharge pour permettre la chute d'un objet passant la trappe, on a prévu en aval de la trappe un dispositif de pesage pour trier les objets fournis du transporteur de décharge par rapport au poids.

3. Un appareil selon la revendication 1 ou 2, caractérisé en ce que la trappe comprend un clapet (26) prévu dans la piste de décharge d'objets, lequel clapet est attaché, au bout d'amont, rotativement á un châssis ou pareil.

4. Un appareil selon la revendication 1, caractérise en ce qu'on a prévu un dispositif de "saut de lapin" (13) en aval de la trappe.

5. Un appareil selon la revendication 1, caractérisé en ce que la trappe comprend un organe en fourche (7) adapté pour mouvement vertical, dont les braches peuvent passer, pendant leur mouvement, le long des dents d'une plaque guide (10) arrangée à angle aigu par rapport à la verticale.

6. Un appareil selon la revendication 5, caractérise en ce que la plaque guide (10) est raccordée à un bac de décharge (11).

7. Un appareil selon l'une quelconque des revendications précédentes, caractérise en ce que des plaques de transfert sont prévues entre les divers transporteurs arrangés dans la piste des objets.

FIG.1

FIG.2

FIG.3